# EUROPÄISCHE PATENTSCHRIFT

(11) **EP 1 007 970 B1**
(45) Veröffentlichungstag und Bekanntmachung des Hinweises auf die Patenterteilung: **13.10.2004**
(21) Anmeldenummer: 98943805.6
(22) Anmeldetag: 30.07.1998
(51) Int. Cl.: G01N 33/569, G01N 33/574, C07K 14/025

(54) **DIAGNOSEKIT FÜR HAUTTEST UND VERFAHREN ZUR DURCHFÜHRUNG DESSELBEN**
DIAGNOSTIC KIT FOR SKIN TESTS, AND METHOD
TROUSSE DE DIAGNOSTIC POUR TESTS CUTANES, ET METHODE APPLICABLE

(30) Priorität: 27.08.1997 DE 19737409
(43) Veröffentlichungstag der Anmeldung: 14.06.2000
(73) Patentinhaber: Medigene Aktiengesellschaft Gesellschaft für Molekularbiologische Kardiologie und Onkologie, 82152 Martinsried (DE)
(72) Erfinder: HÖPFL, Reinhard, Universitätsklinik für, A-6020 Innsbruck (AT)
(74) Vertreter: Leidescher, Thomas, Dr.
(86) Internationale Anmeldenummer: PCT/EP1998/004773
(87) Internationale Veröffentlichungsnummer: WO 1999/010744

(56) Entgegenhaltungen:
- EP-A- 0 386 734
- EP-A- 0 451 550
- DE-U- 9 106 105
- MEDLINE, Washington DC USA; abstract no. 91073133; see abstract XP002090517 & M. MULLER ET AL.: "Identification ofseroreactive regions of the human papillomavirus type 16 protein E4, E6, E7 and L1" JOURNAL OF GENERAL VIROLOGY, Bd. 71, 1990, Seiten 2709-2717, Oxford UK
- M. Nimako et al, Cancer Res vol. 57, 1997, S. 4855-4861

## Beschreibung

Die vorliegende Erfindung betrifft einen Hauttest zum Feststellen des Immunstatus gegen humane Papillomaviren.

Humane Papillomaviren (HPV) sind mit malignen Karzinomen des Anogenitaltrakts, speziell des Cervix uteri, der Vulva, des Penis und des Analkanals, eng verbunden. "High-risk" HPV Typen - wie z.B. HPV16 - transformieren Keratinozyten mittels der in ihnen enthaltenen viralen Onkoproteine E6 und E7 durch Inaktivierung von Tumorsuppressor-Proteinen. Hierbei wird davon ausgegangen, daß die beiden Onkoproteine E6 und E7 kooperativ an der Immortalisierung beteiligt sind (Hawley-Nelson, EMBO J., 8, 3905 (1989)).

Die durch HPV transformierten Krebszellen exprimieren auch noch in fortgeschrittenen Tumorstadien die viralen Proteine E6 und E7. Es wird vermutet, daß eine spontane oder eine durch Impfung induzierte Immunreaktion gegen diese Proteine zur Rückbildung von durch HPV bedingten Tumoren führen könnte. Bisher wurden Patienten oder Patientinnen mit spontaner Rückbildung ihrer Erkrankung nicht untersucht, weil Untersuchungen der zellulären Abwehrreaktion *in vitro* äußerst zeitaufwendig und trotzdem relativ wenig empfindlich sind. Die Rolle der zellulären Immunantwort bei Impfungen gegen HPV-assoziierte Läsionen ist unklar.

Hauttestungen werden teilweise bereits im klinischen Bereich in der Allergologie und bei Infektionskrankheiten (in erster Linie bei der Abklärung der Tuberkulose in Form des bekannten TINE-Tests) eingesetzt. Für virologische Fragestellungen oder für die Krebsforschung gab es jedoch bisher praktisch keine spezifische Anwendung.

Hauttestungen sind auch für wissenschaftliche Fragestellungen ein hervorragendes Werkzeug und zur raschen und praktikablen Erfassung komplexer zellulärer Immunreaktionen *in vivo* geeignet. Ein Hauttest zur Untersuchung der zellulären Immunreaktion gegen "high risk" HPV ist im Stand der Technik vorbekannt. Diese erste Anwendung des Hauttests zur Feststellung einer Immunreaktion gegen ein krebsassoziiertes Virus wurde mit dem Hüllprotein L1 des "high risk" HPV Typ 16 durchgeführt (Gebrauchsmuster G9106105.9) und in der Folge in Tiermodellen angewandt.

Ein Modell für den Hauttest gibt es auch bei der Maus; es wurde dabei die Immunreaktion auf ein transplantiertes Antigen untersucht (McLean et al. J. Gen. Virol. **74**, 239-245 (1993)).

Dieser vorbekannte Hauttest erfaßte nicht die humorale Immunantwort auf eine HPV-Infektion. Im Gegensatz zur zellulären Abwehr ist die Erfassung der humoralen Immunreaktion relativ einfach im Labor mittels serologischer Tests durchführbar. Es gilt als bewiesen, daß Antikörper gegen dreidimensional intakte Strukturen der Virushüllproteine Papillomaviren neutralisieren können (Christensen et al. J Gen Virol **75**, 2271 (1994)) . Antikörper gegen die frühen Proteine E6 und E7 sind mit malignen Tumoren der Cervix uteri assoziiert (Jochmus-Kudielka et al. J. Natl. Cancer Inst. 81, 1698 (1989)).

Eine prophylaktische Impfung mit aus Hüllproteinen bestehenden "virus like particles" würde eine HPV-Infektion und das damit verbundene Krebsrisiko vorbeugend reduzieren. Die humorale Immunantwort gegen HPV spielt aber keine Rolle bei der Rückbildung von bereits bestehenden Läsionen. Die Serologie hat daher epidemiologische Bedeutung.

Zusammenfassend zeigen die Experimente in Tiermodellen und bei Patient innen, daß zelluläre Immunreaktionen gegen virale Proteine durch Hauttestung nachweisbar sind. Aufgrund der Daten kann vermutet werden, daß eine Immunreaktion gegen das Hüllprotein L1 nicht mit Regression einhergeht, da in fortgeschrittenen Läsionen intakte Viruspartikel nicht mehr gebildet werden. Daher sind die transformierenden Virusproteine E6 und E7 das wahrscheinliche Ziel einer "heilenden" Immunreaktion. Diese Tumorantigene sind somit potentielle Kandidaten für die Entwicklung eines Impfstoffs, wenn zusätzlich zur prophylaktischen Wirksamkeit durch Virushüllproteine eine therapeutische Effektivität erzielt werden soll. Es wird angenommen, daß durch eine solche Impfung zytotoxische T-Zellen und T-Helferzellen gegen persistent infizierte Genitalläsionen aktiviert werden.

Ein in vivo Test, um den Erfolg einer solchen Impfung gegen E6 und/oder B7 anhand der vom Immunsystem als Reaktion auf die Impfung gezeigten zellulären Abwehrreaktion nachzuweisen, ist jedoch bislang nicht bekannt.

Aus der EP-A-0 386 734 sind zwei immunogene Regionen des E7-Proteins des Humanpapillomavirus vom Typ 16 (HPV 16) bekannt, wobei die eine immunreaktive Region stromabwärts von Nukleotid 595 und die andere immunreaktive Region stromabwärts von Nukleotid 667 des HPV 16-Genoms lokalisiert ist.

Die EP-A-0 451 550 betrifft seroaktive Epitope der Humanpapillomavirus (HPV) 16-Proteine E4, E6, E7 und L1 sowie Peptide, die solche Epitope enthalten, und die Verwendung dieser Peptide zur Herstellung eines Impfstoffs und eines Diagnosekits.

Aufgabe der Erfindung ist daher die Bereitstellung eines Tests, mit dem der Immunstatus gegen B6- und/oder E7-Proteine von Papillomaviren *in vivo* auf einfache Weise festgestellt werden kann. Diese Aufgabe wird erfindungsgemäß durch die Bereitstellung des Diagnosekits für Hauttests gemäß dem unabhängigen Patentanspruch 1 sowie die Verwendüng gemäß dem unabhängigen Patentanspruch 10 gelöst. Weitere vorteilhafte Ausgestaltungen und Aspekte der Erfindung ergeben sich aus den abhängigen Ansprüchen und der Beschreibung.

Der erfindungsgemäße Hauttest kann auch zum Nachweis spontaner Regressionen von Papillomaviruskarzinomvorläufern (CIN) eingesetzt werden.

In einem weiteren Aspekt kann der erfindungsgemäße Hauttest auch im Bereich der Forschung eingesetzt werden, um Mechanismen bei der Rückbildung von durch HPV bedingten Läsionen zu untersuchen.

Im folgenden wird die vorliegende Erfindung anhand eines Beispiels erläutert, bei dem die prinzipielle Eignung des Hauttests für die Überprüfung von Impferfolgen anhand von nicht geimpften Patientinnen, die jedoch zum Teil spontane Regressionen der Zervixkarzinome und Vorläufer derselben, und somit eine dem Impferfolg gleichzusetzende Eigenschaft aufwiesen, gezeigt wird. Es wurden synthetische Peptide für den Hauttest gegen HPV16-E6 oder -E7 verwendet. Diese bieten gegenüber der Verwendung von nativen Proteinen oder Fusionsproteinen den Vorteil, daß eine Verunreinigung mit DNA ausgeschlossen werden konnte, da synthetische Peptide mit absoluter Sicherheit frei von problematischer Kontamination mit DNA sind.

Eine beispielhafte Hauttestung mit den Peptidantigenen für das Onkoprotein HPV16-E7 wurde bei Patientinnen durchgeführt. Es konnte überraschend gezeigt werden, daß erstens eine zelluläre Immunreaktion gegen das virale Onkoprotein HPV16-E7 *in vivo* erfaßbar ist und zweitens synthetische Peptide Proteine für Hauttestungen ersetzen können. Drittens konnte eine Assoziation einer positiven Hauttestreaktion im Sinne einer "delayed type hypersensitivity" mit Regression zervikaler Krebsvorläufer beobachtet werden. Eine solche Korrelation ist nicht vorbekannt. Die Beobachtung untermauert die potentielle Bedeutung des Antigens HPV16-E7 in einem therapeutisch wirksamen Impfstoff und zeigt die Verwendbarkeit des erfindungsgemäßen Hauttests für die Bestimmung des Immunstatus bezüglich E6 und E7.

Für die Ausführung des erfindungsgemäßen Beispiels wurden fünf einander überlappende, relativ lange Peptide aus je ca. 30 Aminosäuren für das Onkoprotein HPV16-E7 und ein Kontrollpeptid zufälliger Sequenz gleicher Länge verwendet. Die Sequenz für die 98 Aminosäuren des HPV16-E7-Proteins ist bekannt (Seedorf et al. J. Gen. Virol., **71**, 2719 (1990)). Die Peptide wurden am Dep. IHB/AZL (Leiden, Holland) in einem "multiple peptide synthesizer" hergestellt. Die Peptide wurden mit Hilfe der "Fmoc"-Technologie synthetisiert, mittels Äther aus Trifluorsäure präzipitiert und anschließend lyophilisiert. Die Reinheit der Peptide wurde mittels "reverse phase high pressure liquidchromatography" überprüft. Peptide zum Einsatz mit dem erfindungsgemäßen Hauttest können jedoch auch auf andere, Fachleuten geläufige Weise hergestellt werden.

Die im Einbuchstaben-Code angegebenen Sequenzen für die einzelnen getesteten Peptide waren:
1. MHGDTPTLHEYMLDLQPETTDLYCYEQLND
2. DLYCYEQLNDSSEEEDEIDGPAGQAEPDRA
3. PAGQAEPDRAHYNIVTFCCKCDSTLRLCVQ
4. CDSTLRLCVQSTHVDIRTLEDLLMGTLGIV
5. STHVDIRTLEDLLMGTLGIVCPICSQKP
sowie als Kontrollpeptid:
6. SENKELKKAIDGLQGLLLGLRQRIETLEGK

Der Einbuchstaben-Code der Aminosäuren ist beispielsweise in Römpps Chemie Lexikon, Georg Thieme Verlag, Stuttgart, Band 1, Seite 160/161, (1989) erläutert.

Für die Hauttestung wurden die Peptide nach Sterilitätsüberprüfung und Pyrogentest in einer Konzentration von 1 mg/ml in 70% Glycerin gelöst und etwa 0,01 ml dieser Lösung streng intrakutan in die oberste Dermis und die Epidermis der Testpersonen injiziert. Jedes Peptid wurde getrennt (5 HPV16-E7-Peptide + 1 Kontrollpeptid) eingesetzt. Es versteht sich jedoch, daß auch Kombinationen der Peptide untereinander oder/und mit entsprechenden Peptiden von HPV E6 verwendet werden können. Parallel zur Hauttestung mit E7-Antigenen wurde zumeist die allgemeine Abwehrlage gegen klassische Recall-Antigene mit einem Immunblock, z.B. Mérieux-Multitest "Sero", Testsystem mit 7 Antigenen und einer Kontrolle zur Bestimmung des Status der zellvermittelten Immunität, hergestellt vom serotherapeutischen Institut Wien GmbH in Lizenz von Pasteur Merieux S.V., Lyon, Frankreich, bestimmt. Als positiv wurden Reaktionen mit einer rötlichen Papelbildung mit mindestens 2 mm Durchmesser gewertet.

Die Hauttestung wurden an insgesamt 19 Patientinnen mit prämalignen oder malignen HPV bedingten Läsionen ausgeführt. Solche Erkrankungen sind mit einer Wahrscheinlichkeit von in etwa 50% durch den HPV-Typ 16 verursacht, in weiteren 40% der Fälle sind andere "high risk"-HPV-Typen anzunehmen:
Zwölf Patientinnen mit höhergradigen zervikalen intraepithelialen Neoplasien (ZIN III), davon 4 mit Regressionshinweis zum Zeitpunkt der Untersuchung (spontaner Rückgang von Pap III D auf Pap II)
Sieben Patientinnen mit Zevixkarzinomen.

Somit wurden 19 Patientinnen getestet, von denen 4 Regressorinnen und 15 Progressorinnen waren.

Als Kontrollpersonen wurden zwei Männer ohne Hinweis auf HPV16-Infektion verwendet.

Begleitend wurden die hautgetesteten Personen serologisch mittels ELISA mit "virus like partikles (VLPs)" als Antigen auf neutralisierende Antikörper gegen HPV 16 in Fachleuten geläufiger Art und Weise untersucht. Serologische Untersuchungen sind für den spezifischen Hauttest selbst nicht nötig, wurden jedoch im Beispiel zur Erweiterung der wissenschaftlichen Aussagekraft der Ergebnisse mit durchgeführt. Abstrich- oder Biopsiematerial der Läsionen wurde für spätere Virustypisierung mittels PCR bei -80°C eingelagert, vorerst wurde mit dot blot (ViraType, Digene Diagnostics, Silver Spring, MD) auf die HPV-6/11, -16/18 und - 31/33/35 untersucht. Eine Lymphozytenbank für ergänzende in vitro Untersuchungen wurde angelegt.

Die Regressorinnen (4/4) zeigten eine mäßige (1 Fall) bis starke (3 Fälle) Immunreaktion gegen einzelne Peptide des Onkoproteins E7. Bei den 15 Progressorinnen und bei beiden Kontrollpersonen fand sich keine eindeutige Reaktivität im Hauttest. Der Verlauf der Hauttestreaktion war im Vergleich zu einer klassischen Tuberkulinreaktion deutlich verzögert. Ablesungen waren deshalb meist eine Woche nach der Testung am besten möglich. Reaktionen auf das Kontrollpeptid fanden sich in keinem Fall.

Die Reaktionen wurden fotodokumentiert, eine Reaktion wurde biopsiert und histologisch untersucht. Ähnlich wie schon bei früheren Hauttestungen mit L1 beobachtet, fand sich ein lymphozytäres Infiltrat, welches mit einer "delayed type hypersensitivity reaction" vereinbar erschien. Antikörper gegen HPV16-L1 fanden sich in 4 der 7 Patientinnen mit Zervixkarzinom und bei nur einer Patientin mit CIN. Zusammenfassend ging somit der Antikörpernachweis gegen HPV16-L1-VLPs offensichtlich eher mit einer Progression einher, während eine zelluläre Immunreaktion gegen HPV16-E7 im Hauttest mit der Rückbildung präkanzeröser zervikaler Läsionen assoziiert war.

Die Ergebnisse belegen die Wirksamkeit des Hauttests zum empfindlichen Nachweis einer zellulären Immunreaktion gegen HPV16-E7, die bei den untersuchten Fällen mit spontaner Rückbildung einer HPV-assoziierten Krebsvorläuferläsion einherging.

Da mit dem beschriebenen Hauttest eine Immunreaktion erfaßt wurde, die mit Regression von Krebsvorläuferläsionen assoziiert war, kann der Hauttest als Instrument zur Kontrolle eines Impferfolges gegen E6 und/oder E7 eingesetzt werden. Ein breit angelegtes Screening mittels Hauttestung zur Aufklärung der Mechanismen einer immunologisch mediierten Tumorrückbildung ist ebenfalls möglich. Aufgrund des extrem hohen Aufwandes für *in vitro* Experimente ist die Untersuchung einer zellulären Immunität bei einer großen Zahl von Patienten durch Laboruntersuchungen kaum möglich. Im Rahmen zukünftiger Impfstudien mit einer hypothetisch therapeutisch wirksamen Vakzine gegen das Zervixkarzinom durch Immunisierung gegen das Tumorprotein HPV16-E7 ist der erfindungsgemäße Hauttest eine ideale, weil praktikable und sensitive Methode, um das gewünschte Ansprechen des zellulären Immunsystems auf die Impfung zu erfassen.

Im obigen Beispiel wurden die verwendeten Peptide in 70% Glycerin gelöst. Glycerin ist bevorzugt, da es eine für die intrakutane Applikation geeignete Viskosität aufweist und zudem desinfizierend wirkt. Erfindungsgemäß können jedoch auch andere geeignete Lösungsmittel verwendet werden, unter Umständen muß aufgrund des Löseverhaltens eines Peptids dieses in einer speziell angepaßten Lösung gelöst werden. Den erfindungsgemäß verwendeten Lösungsmitteln können auch weitere Additive zugesetzt sein, wie Emulgatoren, Chelatoren, Desinfektionsmittel u.a.

Der Hauttest kann durch intrakutane Injektion einer wirksamen Menge an gelöstem E6- und/oder E7-Antigen mittels einer Spritze durchgeführt werden. Demzufolge enthielte das Diagnosekit eine oder mehrere Ampullen, Spritzen und Kanülen. Erfindungsgemäß ist auch die Verwendung von speziell für Hauttestungen entwickelten Applikatoren möglich und bevorzugt, wie etwa der Multitest "SERO"-Teststempel von Sero-Merieux (siehe oben) oder der Stempel für den TINE-Test. Das erfindungsgemäße Diagnosekit kann demzufolge Ampullen mit Antigen(en) und/oder Applikatoren enthalten.

Im obigen Beispiel wurde anhand von HPV16-E7 die Wirkungsweise des erfindungsgemäßen Hauttests beschrieben. Es kann jedoch auch E6 verwendet werden. Ebenso ist es möglich, den Hauttest durch die Verwendung von E6 und/oder E7 anderer HPV-Typen zur Erkennung einer Immunantwort gegenüber diesen Stämmen einzusetzen. Durch den nahen Verwandschaftsgrad verschiedener HPV-Typen ist es schließlich auch möglich, Kreuzreaktionen zwischen verschiedenen HPV-Typen auszunutzen, um einen universelleren Hauttest gegen verschiedene HPV-Typen gleichzeitig einzuführen.

Die für eine sichtbare Immunreaktion notwendig applizierte Menge von Antigenen hängt von verschiedenen Faktoren ab. Sie kann daher erfindungsgemäß zwischen 0,01 und 10 µg, vorzugsweise zwischen 0,05 und 5 µg Antigen pro Applikation liegen. Hierbei ist für die Zusammenstellung von Antigenlösungen für Diagnosekits zu beachten, daß nur ein Teil der Lösung in den intrakutanen Bereich gelangt, während ein anderer Teil auf der Haut des Probanden verlorengehen kann oder in der Lösungsampulle zurückbleibt.

## Patentansprüche

1. Hauttest-Diagnosekit zum Nachweis einer zellulären Immunreaktion gegen das Onkoprotein E6 und/oder E7 eines Humanpapillomavirentyps, wobei das Diagnosekit eine wirksame Menge des Onkoproteins E6 und/oder E7 und/oder mindestens eines immunologisch wirksamen Teils von E6 und/oder E7 eines Humanpapillomavirustyps sowie einen Applikator, mit dem die wirksame Menge des Onkoproteins oder des immunologisch wirksamen Teils desselben intrakutan applizierbar ist, enthält.

2. Diagnosekit gemäß Anspruch 1, **dadurch gekennzeichnet, daß** der enthaltene Teil des Onkoproteins aus HPV 16 stammt.

3. Diagnosekit gemäß Anspruch 1 oder 2, **dadurch gekennzeichnet, daß** der immunologisch wirksame Teil des Humanpapillomavirustyps mindestens ein synthetisch hergestelltes Peptid ist.

4. Diagnosekit gemäß einem der vorhergehenden Ansprüche, **dadurch gekennzeichnet, daß** das enthaltene Onkoprotein E7 oder der immunologisch wirksame Teil desselben aus HPV 16 ist oder sind.

5. Diagnosekit gemäß einem der vorhergehenden Ansprüche, **dadurch gekennzeichnet, daß** das enthaltene Onkoprotein oder der immunologisch wirksame Teil desselben in einem Lösungsmittel gelöst ist.

6. Diagnosekit gemäß Anspruch 5, **dadurch gekennzeichnet, daß** das Lösungsmittel 70% Glycerin ist.

7. Diagnosekit gemäß einem der vorhergehenden Ansprüche, **dadurch gekennzeichnet, daß** die Menge Onkoprotein oder des immunologisch wirksamen teils 0,01 bis 10 µg pro zu applizierende Charge beträgt.

8. Diagnosekit gemäß Anspruch 1, **dadurch gekennzeichnet, daß** der Applikator eine Spritze ist.

9. Diagnosekit gemäß Anspruch 1, **dadurch gekennzeichnet, daß** der Applikator ein Teststempel ist, wie er beispielsweise für den TINE-Test oder den Multitest "Sero" zum Einsatz kommt.

10. Verwendung des Onkoproteins E6 und/oder E7 und/oder mindestens eines immunologisch wirksamen Teils von E6 und/oder E7 eines Humanpapillomavirustyps zur Herstellung eines Diagnosekits gemäß einem der Ansprüche 1-9 zum Nachweis einer zellulären Immunreaktion gegen das Onkoprotein E6 und/oder E7 eines Humanpapillomavirustyps.

11. Verwendung gemäß Anspruch 10, **dadurch gekennzeichnet, daß** der enthaltene Teil des Onkoproteins aus HPV16 stammt.

12. Verwendung gemäß Anspruch 10 oder 11, **dadurch gekennzeichnet, daß** der immunologisch wirksame Teil des Humanpapillomavirustyps mindestens ein synthetisch hergestelltes Peptid ist.

13. Verwendung gemäß einem der Ansprüche 10 bis 12, **dadurch gekennzeichnet, daß** das enthaltene Onkoprotein E7 oder der immunologisch wirksame Teil desselben aus HPV16 ist oder sind.

14. Verwendung gemäß einem der Ansprüche 10 bis 13, **dadurch gekennzeichnet, daß** das enthaltene Onkoprotein oder der immunologisch wirksame Teil desselben in einem Lösungsmittel gelöst ist.

15. Verwendung gemäß Anspruch 14, **dadurch gekennzeichnet, daß** das Lösungsmittel 70% Glycerin ist.

16. Verwendung gemäß einem der Ansprüche 10 bis 15, **dadurch gekennzeichnet, daß** die Menge Onkoprotein oder des immunologisch wirksamen Teils 0,01 bis 10 µg pro zu applizierende Charge beträgt.

17. Verwendung gemäß Anspruch 10, **dadurch gekennzeichnet, daß** das Diagnosekit weiterhin einen Applikator umfaßt, mit dem die wirksame Menge des Onkoproteins oder des immunologisch wirksamen Teils desselben intrakutan applizierbar ist.

18. Verwendung gemäß Anspruch 17, **dadurch gekennzeichnet, daß** der Applikator eine Spritze ist.

19. Verwendung gemäß Anspruch 17, **dadurch gekennzeichnet, daß** der Applikator ein Teststempel ist, wie er beispielsweise für den TINE-Test oder den Multitest "Sero" zum Einsatz kommt.

## Claims

1. A skin test diagnosis kit for proving a cellular immune reaction against the oncoprotein E6 and/or E7 of a human papilloma virus type, the diagnosis kit containing an effective amount of the oncoprotein E6 and/or E7 and/or at least an immunologically effective part of E6 and/or E7 of a human papilloma virus type, and an applicator, with which the effective amount of the oncoprotein or the immunologically effective part thereof can be applied intracutaneously.

2. The diagnosis kit according to claim 1, **characterized in that** the part of the oncoprotein contained is from HPV16.

3. The diagnosis kit according to claim 1 or 2, **characterized in that** the immunologically effective part of the human papilloma virus type is at least one synthetically produced peptide.

4. The diagnosis kit according to one of the preceding claims, **characterized in that** the oncoprotein E7 contained or the immunologically effective part thereof is or are from HPV16.

5. The diagnosis kit according to one of the preceding claims, **characterized in that** the oncoprotein contained or the immunologically effective part thereof is dissolved in a solvent.

6. The diagnosis kit according to claim 5, **characterized in that** the solvent is 70% glycerin.

7. The diagnosis kit according to one of the preceding claims, **characterized in that** the amount of oncoprotein or the immunologically effective part is 0.01 to 10 µg per charge to be applied.

8. The diagnosis kit according to claim 1, **characterized in that** the applicator is a syringe.

9. The diagnosis kit according to claim 1, **characterized in that** the applicator is a test stamp, as is for example used for the TINE-Test or the multitest "Sero".

10. Use of the oncoprotein E6 and/or E7 and/or at least of an immunologically effective part of E6 and/or E7 of a human papilloma virus type for the production of a diagnosis kit according to one of claims 1 to 9 for proving a cellular immune reaction against the oncoprotein E6 and/or E7 of a human papilloma virus type.

11. Use according to claim 10, **characterized in that** the part of the oncoprotein contained is from HPV16.

12. Use according to claim 10 or 11, **characterized in that** the immunologically effective part of the human papilloma virus type is at least one synthetically produced peptide.

13. Use according to one of claims 10 to 12, **characterized in that** the oncoprotein E7 contained or the immunologically effective part thereof is or are from HPV16.

14. Use according to one of claims 10 to 13, **characterized in that** the oncoprotein contained or the immunologically effective part thereof is dissolved in a solvent.

15. Use according to claim 14, **characterized in that** the solvent is 70% glycerin.

16. Use according to one of claims 10 to 15, **characterized in that** the amount of oncoprotein or the immunologically effective part is 0.01 to 10 µg per charge to be applied.

17. Use according to claim 10, **characterized in that** the diagnosis kit comprises furthermore an applicator, with which the effective amount of the oncoprotein or of the immunologically effective part thereof can be applied intracutaneously.

18. Use according to claim 17, **characterized in that** the applicator is a syringe.

19. Use according to claim 17, **characterized in that** the applicator is a test stamp, as is for example used for the TINE-Test or the multitest "Sero".

## Revendications

1. Kit de diagnostic pour tests cutanés permettant de déceler une réaction immunitaire cellulaire à l'oncoprotéine E6 et/ou E7 d'un type de papilloma virus humain (HPV), ledit kit de diagnostic comprenant une quantité active d'oncoprotéine E6 et/ou E7 et/ou au moins une partie immunologiquement active d'E6 et/ou E7 d'un type de papilloma virus humain ainsi qu'un applicateur grâce auquel la quantité active de l'oncoprotéine ou de la partie immunologiquement active de cette dernière peut être appliquée de manière intracutanée.

2. Kit de diagnostic selon la revendication 1, **caractérisé en ce que** la partie contenue de l'oncoprotéine provient d'un papilloma virus humain de type 16 (HPV16).

3. Kit de diagnostic selon la revendication 1 ou 2, **caractérisé en ce que** la partie immunologiquement active du type de papilloma virus humain est au moins un peptide synthétisé.

4. Kit de diagnostic selon l'une des revendications précédentes, **caractérisé en ce que** l'oncoprotéine E7 contenue ou la partie immunologiquement active de celle-ci provient ou proviennent d'un HPV16.

5. Kit de diagnostic selon l'une des revendications précédentes, **caractérisé en ce que** l'oncoprotéine contenue ou la partie immunologiquement active de celle-ci est dissoute dans un solvant.

6. Kit de diagnostic selon la revendication 5, **caractérisé en ce que** le solvant est composé à 70% de glycérine.

7. Kit de diagnostic selon l'une des revendications précédentes, **caractérisé en ce que** la quantité d'oncoprotéine ou de la partie immunologiquement active est de 0,01 à 10 µg par charge applicable.

8. Kit de diagnostic selon la revendication 1, **caractérisé en ce que** l'applicateur est une seringue.

9. Kit de diagnostic selon la revendication 1, **caractérisé en ce que** l'applicateur est un tampon de test tel qu'utilisé, par exemple, dans le TINE test ou dans le multitest "séro".

10. Utilisation de l'oncoprotéine E6 et/ou E7 et/ou d'au moins une partie immunologiquement active d'E6 et/ou d'E7 d'un type de papilloma virus humain permettant la réalisation d'un kit de diagnostic, selon l'une des revendications 1 à 9, afin de déceler une réaction immunitaire cellulaire à l'oncoprotéine E6 et/ou E7 d'un type de papilloma virus humain.

11. Utilisation selon la revendication 10, **caractérisée en ce que** la partie contenue de l'oncoprotéine provient d'un papilloma virus humain de type 16 (HPV16).

12. Utilisation selon la revendication 10 ou 11, **caractérisée en ce que** la partie immunologiquement active du type de papilloma virus humain est au moins un peptide synthétisé.

13. Utilisation selon l'une des revendications 10 à 12, **caractérisée en ce que** l'oncoprotéine E7 contenue ou la partie immunologiquement active de celle-ci provient ou proviennent d'un HPV16.

14. Utilisation selon l'une des revendications 10 à 13, **caractérisée en ce que** l'oncoprotéine contenue ou la partie immunologiquement active de celle-ci est dissoute dans un solvant.

15. Utilisation selon la revendication 14, **caractérisée en ce que** le solvant est composé à 70% de glycérine.

16. Utilisation selon l'une des revendications 10 à 15, **caractérisée en ce que** la quantité d'oncoprotéine ou de la partie immunologiquement active est de 0,01 à 10 µg par charge applicable.

17. Utilisation selon la revendication 10, **caractérisée en ce que** le kit de diagnostic comprend en outre un applicateur grâce auquel la quantité active de l'oncoprotéine ou de la partie immunologiquement active de cette dernière peut être appliquée de manière intracutanée.

18. Utilisation selon la revendication 17, **caractérisée en ce que** l'applicateur est une seringue.

19. Utilisation selon la revendication 17, **caractérisée en ce que** l'applicateur est un tampon de test tel qu'utilisé, par exemple, dans le TINE test ou dans le multitest "séro".


### SEQUENZPROTOKOLL

(1) ALLGEMEINE ANGABEN:
   (i) ANMELDER:
      (A) NAME: MediGene AG
      (B) STRASSE: Lochhamer Str. 11
      (C) ORT: Muenchen
      (D) BUNDESLAND: Bayern
      (E) LAND: Deutschland
      (F) POSTLEITZAHL: 81927
      (G) TELEFON: +49-89-8956320
      (H) TELEFAX: +49-89-89563220

      (A) NAME: Dr. Reinhard Hoepfl
      (B) STRASSE: Anichstr. 35
      (C) ORT: Innsbruck
      (E) LAND: Oesterreich
      (F) POSTLEITZAHL: 6020
   (ii) BEZEICHNUNG DER ERFINDUNG: Diagnosekit fuer Hauttest und Verfahren zur Durchfuehrung desselben
   (iii) ANZAHL DER SEQUENZEN: 6
   (iv) COMPUTER-LESBARE FASSUNG:
      (A) DATENTRAGER: Floppy disk
      (B) COMPUTER: IBM PC compatible
      (C) BETRIEBSSYSTEM: PC-DOS/MS-DOS
      (D) SOFTWARE: PatentIn Release #1.0, Version #1.30 (EPA)
(2) ANGABEN ZU SEQ ID NO: 1:
   (i) SEQUENZKENNZEICHEN:
      (A) LANGE: 30 Aminosäuren
      (B) ART: Aminosäure
      (C) STRANGFORM:
      (D) TOPOLOGIE: nicht bekannt
   (ii) ART DES MOLEKÜLS: Peptid
   (iii) HYPOTHETISCH: NEIN
   (iv) ANTISENSE: NEIN
   (v) ART DES FRAGMENTS: inneres Fragment
   (xi) SEQUENZBESCHREIBUNG: SEQ ID NO: 1:
   Met His Gly Asp Thr Pro Thr Leu His Glu Tyr Met Leu Asp Leu Gln
   1               5                   10                  15
   Pro Glu Thr Thr Asp Leu Tyr Cys Tyr Glu Gln Leu Asn Asp
               20                  25                  30
(2) ANGABEN ZU SEQ ID NO: 2:
   (i) SEQUENZKENNZEICHEN:
      (A) LANGE: 30 Aminosäuren
      (B) ART: Aminosäure
      (C) STRANGFORM:
      (D) TOPOLOGIE: nicht bekannt
   (ii) ART DES MOLEKULS: Peptid
   (iii) HYPOTHETISCH: NEIN
   (iv) ANTISENSE: NEIN
   (v) ART DES FRAGMENTS: inneres Fragment
   (xi) SEQUENZBESCHREIBUNG: SEQ ID NO: 2:
   Asp Leu Tyr Cys Tyr Glu Gln Leu Asn Asp Ser Ser Glu Glu Glu Asp
   1               5                   10                  15
   Glu Ile Asp Gly Pro Ala Gly Gln Ala Glu Pro Asp Arg Ala
               20                  25                  30
(2) ANGABEN ZU SEQ ID NO: 3:
   (i) SEQUENZKENNZEICHEN:
      (A) LÄNGE: 30 Aminosäuren
      (B) ART: Aminosäure
      (C) STRANGFORM:
      (D) TOPOLOGIE: nicht bekannt
   (ii) ART DES MOLEKULS: Peptid
   (iii) HYPOTHETISCH: NEIN
   (iv) ANTISENSE: NEIN
   (v) ART DES FRAGMENTS: inneres Fragment
   (xi) SEQUENZBESCHREIBUNG: SEQ ID NO: 3:
   Pro Ala Gly Gln Ala Glu Pro Asp Arg Ala His Tyr Asn Ile Val Thr
   1               5                   10                  15
   Phe Cys Cys Lys Cys Asp Ser Thr Leu Arg Leu Cys Val Gln
               20                  25                  30
(2) ANGABEN ZU SEQ ID NO: 4:
   (i) SEQUENZKENNZEICHEN:
      (A) LÄNGE: 30 Aminosäuren
      (B) ART: Aminosäure
      (C) STRANGFORM:
      (D) TOPOLOGIE: nicht bekannt
   (ii) ART DES MOLEKULS: Peptid
   (iii) HYPOTHETISCH: NEIN
   (iv) ANTISENSE: NEIN
   (v) ART DES FRAGMENTS: inneres Fragment
   (xi) SEQUENZBESCHREIBUNG: SEQ ID NO: 4:
   Cys Asp Ser Thr Leu Arg Leu Cys Val Gln Ser Thr His Val Asp Ile
   1               5                   10                  15
   Arg Thr Leu Glu Asp Leu Leu Met Gly Thr Leu Gly Ile Val
               20                  25                  30
(2) ANGABEN ZU SEQ ID NO: 5:
   (i) SEQUENZKENNZEICHEN:
      (A) LANGE: 28 Aminosäuren
      (B) ART: Aminosäure
      (C) STRANGFORM:
      (D) TOPOLOGIE: nicht bekannt
   (ii) ART DES MOLEKÜLS: Peptid
   (iii) HYPOTHETISCH: NEIN
   (iv) ANTISENSE: NEIN
   (v) ART DES FRAGMENTS: inneres Fragment
   (xi) SEQUENZBESCHREIBUNG: SEQ ID NO: 5:
   Ser Thr His Val Asp Ile Arg Thr Leu Glu Asp Leu Leu Met Gly Thr
   1               5                   10                  15
   Leu Gly Ile Val Cys Pro Ile Cys Ser Gln Lys Pro
               20                  25
(2) ANGABEN ZU SEQ ID NO: 6:
   (i) SEQUENZKENNZEICHEN:
      (A) LÄNGE: 30 Aminosäuren
      (B) ART: Aminosäure
      (C) STRANGFORM:
      (D) TOPOLOGIE: nicht bekannt
   (ii) ART DES MOLEKÜLS: Peptid
   (iii) HYPOTHETISCH: NEIN
   (iv) ANTISENSE: NEIN
   (v) ART DES FRAGMENTS: inneres Fragment
   (xi) SEQUENZBESCHREIBUNG: SEQ ID NO: 6:
   Ser Glu Asn Lys Glu Leu Lys Lys Ala Ile Asp Gly Leu Gln Gly Leu
   1               5                   10                  15
   Leu Leu Gly Leu Arg Gln Arg Ile Glu Thr Leu Glu Gly Lys
               20                  25                  30